Europäisches Patentamt

European Patent Office

Office européen des brevets

(19)

(11) Veröffentlichungsnummer: **0 247 037**
**B1**

(12) **EUROPÄISCHE PATENTSCHRIFT**

(45) Veröffentlichungstag der Patentschrift: **06.06.90**

(51) Int. Cl.⁵: **A 61 F 7/02**

(21) Anmeldenummer: **85906064.2**

(22) Anmeldetag: **03.12.85**

(86) Internationale Anmeldenummer:
**PCT/EP85/00660**

(87) Internationale Veröffentlichungsnummer:
**WO 86/03400 19.06.86 Gazette 86/13**

(54) **PACKUNG FÜR DIE HEILBEHANDLUNG DURCH ENERGIEZUFUHR ODER -ENTZUG.**

(30) Priorität: **03.12.84 DE 3444066**

(43) Veröffentlichungstag der Anmeldung:
**02.12.87 Patentblatt 87/49**

(45) Bekanntmachung des Hinweises auf die
Patenterteilung:
**06.06.90 Patentblatt 90/23**

(84) Benannte Vertragsstaaten:
**AT BE CH FR GB IT LI NL SE**

(56) Entgegenhaltungen:
**DE-A-3 411 357**
**FR-A-1 018 835**
**FR-A-2 171 135**
**FR-A-2 543 669**
**FR-A-2 543 670**
**US-A-2 566 533**
**US-A-2 602 302**
**US-A-3 075 529**
**US-A-3 463 161**

(73) Patentinhaber: **KNEIPP-WERKE KNEIPP-MITTEL-
ZENTRALE LEUSSER & OBERHÄUSSER
Steinbachtal 43
D-8700 Würzburg 1 (DE)**

(72) Erfinder: **HAKSPIEL, Benedikt
Blumenring 24
D-8703 Ochsenfurt (DE)**

(74) Vertreter: **Kraus, Jürgen Helmut, Dipl.-Phys.
et al
c/o Leinweber & Zimmermann Rosental 7
D-8000 München 2 (DE)**

Courier Press, Leamington Spa, England.

**Beschreibung**

Die Erfindung betrifft eine Packung für die Heilbehandlung von Patienten durch Energiezufuhr oder -entzug mit oder ohne Wirkstoffeinwirkung, wobei die Packung eine wasserdichte und abwaschbare, allseits dicht verschlossene temperaturbeständige Außenhülle und einen in der Außenhülle liegenden Temperaturspeicher aufweist, der in einzelne Behälter unterteilt ist, die mit einem Temperaturspeichermedium gefüllt sind und einen Abstand voneinander aufweisen, wobei weiter der Freiraum zwischen dem in sich einen geschlossenen Behälter darstellenden Temperaturspeicher und der Außenhülle mit einem Temperatur-Puffermaterial gefüllt ist.

Die heilende Wirkung von Wärme oder Kälte ist seit langem bekannt. Dabei läßt man die Temperaturdifferenz auf den Patienten bzw. den zu behandelnden Körperteil entweder für sich einwirken oder fügt noch die Einwirkung eines Wirkstoffes hinzu, wie das beispielsweise bei der Fangopackung der Fall ist.

So sind schon Packungen bekannt (DE—C—26 11 928), bei denen ein wiederverwendbarer abwaschbarer und damit hygienischer Temperaturspeicher mit einer Wegwerf-Wirkstoffpackung kombiniert wird.

Als reine Wärme- oder Kälte-Packung ist auch schon eine Packung der eingangs genannten Art bekannt (FR—A—10 18 835) die eine zweifache Hülle aufweist. Im Inneren der Packung liegt zunächst der Temperaturspeicher in Gestalt eines allseits geschlossenen Behälters, der mit einem Temperaturspeichermedium gefüllt ist. Dabei ist der Temperaturspeicher in einzelne Behälter unterteilt, die beispielsweise quadratisch sind und in Reihen oder Zeilen unter Abstand voneinander angeordnet sind. Dadurch ergibt sich ein System von Gelenken zwischen den Behältern, so daß der ganze Temperaturspeicher auf unebenen Körperoberflächen gut angepaßt werden kann.

Der so aufgebaute Temperaturspeicher ist nun bei der bekannten Packung hoch in eine weitere Hülle eingeschweißt, wobei der Freiraum zwischen dem Temperaturspeicher und der Hülle mit einem Temperatur-Puffermaterial gefüllt ist. Dieses kann sich im Freiraum bewegen und gleicht so noch bestehende Oberflächenunebenheiten zwischen dem Temperaturspeicher und der Körperoberfläche gut aus. Gleichzeitig überträgt es aber bei der Wärmepackung die vom Temperaturspeicher abgegebene Energie zur Körperoberfläche bei der Kältepackung die von der Körperoberfläche abgegebene Energie zum Temperaturspeicher.

Man erhält so eine für die Therapie gut geeignete Packung, die nur noch einen Nachteil aufweist: Physiologisch ist es für den Patienten kaum möglich, die Temperatur der Packung auch nach längerer Behandlungsdauer so deutlich zu empfinden, daß er danach das Behandlungsende bestimmen kann, zu dem sich die Wirkung des Temperaturspeichers erschöpft hat. Selbstverständlich ist es möglich, mit einfachen Faustregeln Behandlungszeiten festzulegen. Dies ist jedoch mit einer erheblichen Fehlerquote behaftet, weil Streugrößen, wie die Ausgangstemperatur der Packung, die Oberflächentemperatur des zu behandelnden Körperteils und die Umgebungstemperatur nicht berücksichtigt werden können. Entweder wird also die Packung nicht vollständig ausgenützt oder es wird die Behandlungszeit unnötig ausgedehnt. Man könnte deshalb daran denken, die Behandlung über eine Temperaturmessung zu kontrollieren. Das stellt aber einen zusätzlichen Aufwand dar, erforderte ein weiteres Gerät und kann wegen der notwendigen Zwischenlage eines Thermometers zwischen die Packung und die Körperoberfläche sogar die therapeutische Wirkung vermindern.

Aufgabe der Erfindung ist es deshalb, die bekannte Packung so auszugestalten, daß der Patient mit einfachen Mitteln einen Hinweis erhält, wann die Behandlung beendet werden kann. Die Lösung dieser Aufgabe ist im kennzeichnenden Teil des Anspruchs 1 angegeben. Eine zweckmäßige Ausgestaltung ist im Anspruch 2 gekennzeichnet.

Die bestgeeigneten Temperaturspeichermedien zeichnen sich dadurch aus, daß sie eine bestimmte erwünschte Behandlungstemperatur (bei Paraffin beispielsweise 52°C) über längere Zeit plateauartig halten. Dieses Wärmeplateau ist es, das auch die sinnvolle Behandlungszeit bestimmt.

Ist nämlich die im Temperaturspeicher gespeicherte Energie erschöpft, so fällt dann die Temperatur rasch ab. Umgekehrtes gilt bei der Kältebehandlung. Ist einmal das gesamte Temperaturspeichermaterial geschmolzen, so steigt hier die Temperatur rasch an. Erfindungsgemäß wird nun diese, das Ende der sinnvollen Behandlung anzeigende, rasche Temperaturänderung nach Verlassen des Temperaturplateaus als Indikator für den Patienten benützt, die Behandlung tatsächlich zu beenden. Hierfür wird ein optischer Anzeiger, wie ein Farbumschlag oder der Übergang von transparent in opak herangezogen. Bei Paraffin kann bei geeigneter Auswahl z.B. erreicht werden, daß das Paraffin im aufgeheizten, flüssigen Zustand transparent ist, aber nach Abgabe der Schmelzwärme im Erstarrungsvorgang opak wird. Voraussetzung für die Erkennbarkeit dieses Vorgangs ist natürlich, daß die Hüllen der Packung durchsichtig sind, so daß die optisch erkennbare Zustandsveränderung im Packungsinneren von außen auch erkannt werden kann.

Weitere Einzelheiten, Vorteile und Merkmale ergeben sich aus der folgenden Beschreibung, in der eine Ausführungsform der erfindungsgemäßen Packung erläutert ist. Es zeigt

Fig. 1 eine Draufsicht auf eine erfindungsgemäße Packung, und

Fig. 2 einen Schnitt durch die Packung von Fig. 1.

Die Packung 10 besteht aus einer äußeren Hülle 12 und einem in diese eingelegten Temperaturspeicher 14. Die Außenhülle besteht aus zwei Polyäthylen-Polyamid-Folien, die längs des Umfangsrandes 16 miteinander verschweißt sind. Selbstverständlich ist es möglich, stattdessen die Außenhülle 12 aus einer einzigen Folie herzustellen, die unter Zwischenlage des Temperaturspeichers 14 auf sich selbst umgeschlagen und dann nur an drei der Außenränder mit sich selbst unter Bildung des Umfangsrandes 16 verschweißt wird, wodurch wieder eine dichte Hülle entsteht.

Zweckmäßig wird die Außenhülle 12 aus einer durchsichtigen Polyäthylen-Polyamid-Folie hergestellt.

Der Temperaturspeicher 14 ist aus ebenfalls durchsichtigem Kunststoff gefertigt, der für die unten noch zu erläuternde Füllung dicht, zugleich aber tiefziehbar sein soll. Ggfls. wird auch mit einer mehrschichtigen Folie aus unterschiedlichen Materialien gearbeitet. Für die Herstellung des in die Hülle 12 eingelegten Temperaturspeichers 14 ist Polyamid geeignet.

Aus dem ausgewählten Material werden zwei Teile hergestellt, nämlich eine Näpfchenplatte 18 und eine Deckfolie 20.

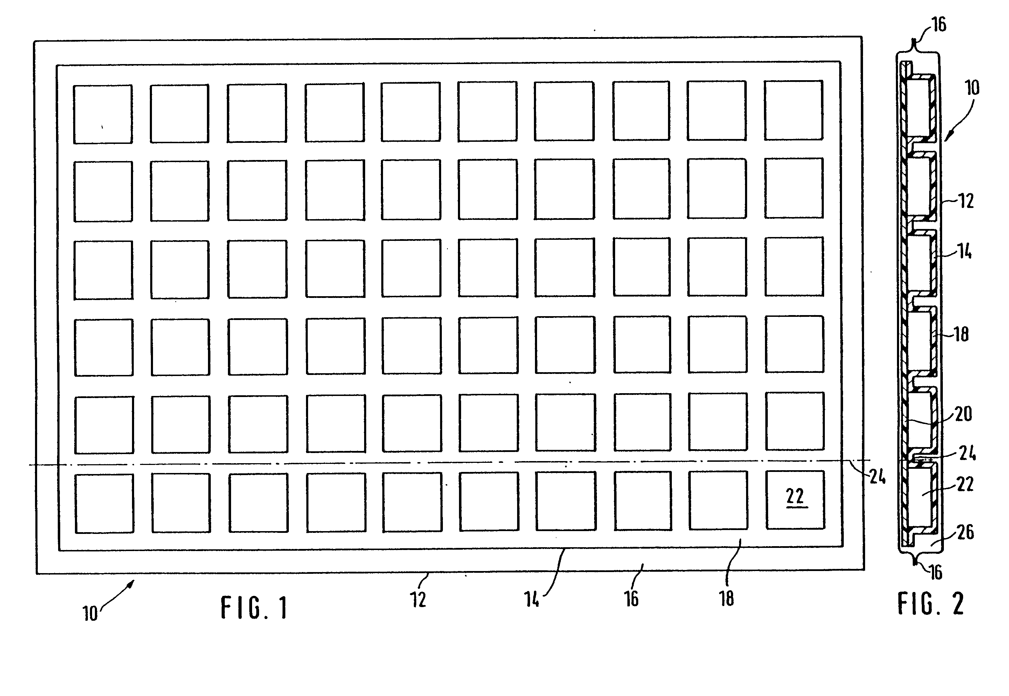

Die Näpfchenplatte 18 besteht aus einer Vielzahl von in Reihen und Zeilen angeordneten Näpfchen 22. Diese sind im gezeigten Ausführungsbeispiel quadratisch mit einer Seitenlänge von 3 cm und einer Tiefe von 10 mm. Die Näpfchen entstehen dadurch, daß eine Folie aus entsprechendem Material durch einen Prägevorgang in diese Form gebracht wird. Dadurch ist gleichzeitig sichergestellt, daß zwischen benachbarten Näpfchen 22 ein sich über nahezu die gesamte Höhe erstreckender Freiraum bleibt. Dieser wirkt, da die Näpfchen in Reihen und Zeilen angeordnet sind, als Biegelinie. Beispielhaft ist eine Biegelinie 24 in die Fig. 1 und 2 eingezeichnet. Da zahlreiche solche Biegelinien im Abstand von 4 cm parallel nebeneinander liegen, ist eine gute Oberflächenanpassung möglich. Dabei sind im gezeigten Ausführungsbeispiel zwei Scharen untereinander paralleler Biegelinien 24 vorgesehen, die aufeinander senkrecht stehen. Wird eine noch bessere Anpassung an die Oberflächen gewünscht, so kann dazu übergegangen werden, mit drei Scharen untereinander paralleler Biegelinien zu arbeiten, die untereinander jeweils Winkel von 60° einschließen. Die Näpfchen 22 haben dann in der Draufsicht die Form gleichseitiger Dreiecke.

Zum Erzielen der Funktion des Temperaturspeichers 14 ist es notwendig, die Näpfchen 22 vor der Anbringung der Deckfolie 20 mit einem entsprechenden Temperaturspeichermedium zu füllen.

Soll die Packung als Wärmepackung eingesetzt werden, so ist hierfür Paraffinum durum gut geeignet. Dieses Material hat seinen Schmelzpunkt bei 52°. Wird es über diese Temperatur aufgeheizt, so wird das Paraffin flüssig und dabei durchsichtig. Bei der Abkühlung ergibt sich ein Wärmeplateau bei 52°C dadurch, daß hier die Schmelzwärme abgegeben wird, bis das Paraffin insgesamt erstarrt und dabei wieder opak geworden ist.

Da ein gewisser Temperaturgradient von der Wärmepackung bis zur Haut des Patienten durch Zwischenlagen eingestellt werden kann, ist durch entsprechende Auswahl der Packung und der Zwischenlagen eine Behandlungstemperatur zwischen 42 und 52°C möglich, die über entsprechend lange Zeit aufrechterhalten werden kann. Dabei wird diese Temperatur gut gleichmäßig über die gesamte Kontaktoberfläche aufrecht erhalten, weil das Paraffin in den einzelnen Näpfchen 22 durch die Deckfolie 20 eingeschlossen ist und so am Verfließen innerhalb des Wärmespeichers gehindert wird. Es steht somit überall die gleiche Wärmekapazität zur Verfügung, so daß auch ohne besondere Aufmerksamkeit des Personals oder des sich selbst behandelnden Patienten gut gleichmäßige Behandlungsergebnisse erzielt werden können.

Ist nach 20 bis 30 Minuten des Paraffin in den Näpfchen 22 erstarrt, so ist das an der Eintrübung bzw. der Farbveränderung des Paraffins leicht zu erkennen.

Um eine Gegenreaktion des Körpers (Wärmeschock) zu vermeiden, der die Wirksamkeit der Wärmeeinwirkung zunächst herabsetzen könnte, kann bei Bedarf die Packung hautseitig mit einem Vlies unterlegt werden. Es empfiehlt sich, dieses Vlies mit der Wärmepackung nicht fest zu verbinden. Die Wärmepackung kann dann leicht hygienisch gereinigt und jeweils über ein neues Vlies mit dem nächsten Patienten in Kontakt gebracht werden.

Wird Wert darauf gelegt, daß die Wärmebehandlung mit einer Wirkstoffeinwirkung kombiniert wird, so kann das Vlies mit einem Wirkstoff bestreut, besprüht oder getränkt sein (bis zu einer Wirkstoffschichtstärke von 20 mm).

Wird nicht eine Wärme-, sondern eine Kältebehandlung gewünscht, so können die Näpfchen 22 beispielsweise mit Wasser gefüllt werden, sofern das Wasser bei einer Änderung seines Aggregatzustandes zwischen fest und flüssig und sichtbare optische Veränderung erfährt. Bei Verwendung von wasser muß für Evakuierung gesorgt werden, um einen Ausdehnungsraum bei Frieren zu erzielen. Die Packung wird vor der Behandlung im Eisschrank unterkühlt, so daß das Wasser in sämtlichen Näpfchen in die Eisphase übergeht. Beim Auflegen der Packung wird auch hier wieder die Temperatur von 0°C über 20 bis 30 Minuten gehalten, bis nämlich durch die dem Patienten entzogene Wärme die gesamte Schmelzwärme aufgebracht ist, die zur Phasenrückumwandlung notwendig ist. Selbstverständlich kann durch entsprechende Zusätze, beispielsweise von Salzen, auch eine andere Behandlungstemperatur (ggfls. weit unter 0°C) gewählt werden.

Der Temperaturspeicher 14 aus der Näpfchenplatte 18 mit den mit dem Temperaturspeichermedium gefüllten Näpfchen 22 und der mit der Oberseite der Näpfchenplatte 18 verschweißten und die Näpfchen abschließenden Deckfolie 20 ist durch seine Konstruktion mit den freien Abständen zwischen den Näpfchenwänden gut beweglich und an die Körperoberfläche anpassungsfähig.

Dies wird aber jetzt noch dadurch verbessert, daß in den Freiraum 26 zwischen der Oberfläche des Temperaturspeichers 14 und der Außenhülle 12 ein Puffermaterial eingefüllt wird. Dieses füllt die Spalten zwischen benachbarten Näpfchen und den Raum zwischen Außenhülle 12 und Oberfläche der Näpfchenplatte 18 aus. Es wird somit zuerst auf die vom Temperaturspeichermedium vorgegebene Temperatur gebracht. Diese Temperatur nimmt das Puffermaterial überall gleichmäßig an und zwar völlig unabhängig von der Näpfchenstruktur der Packung.

Das Puffermaterial hat also eine doppelte Wirkung: Zum einen wird mechanisch dafür gesorgt, daß die kantige Näpfchenstruktur der Näpfchenplatte 18 abgepuffert und ausgeglichen wird und die gesamte Packung eine angenehm nachgiebige Oberfläche erhält. Zum anderen wird die durch die Näpfchenanordnung erzielte gute Verteilung der Temperaturkapazität noch verbessert und ausgeglichen.

Als Puffermaterial ist beispielsweise ein Wassergel geeignet. Dieses wird dadurch erzielt, daß man Wasser mit ca. 3% einen Gelbildner, beispielsweise ein Zellulosederivat und ein Gefrierschutzmittel zusetzt. Dadurch entsteht eine hochviskose Masse, die als Puffermaterial bestens geeignet ist.

**Patentansprüche**

1. Packung für die Heilbehandlung von Patienten durch Energiezufur oder -entzug mit oder ohne Wirkstoffeinwirkung, wobei die Packung (10) eine wasserdichte und abwaschbare, allseits dicht verschlossene temperaturbeständige Außenhülle (12) und einen in der Außenhülle liegenden Temperaturspeicher (14) aufweist, der in einzelne Behälter oder Näpfchen (22) unterteilt ist, die mit einem Temperaturspeichermedium gefüllt sind und einen Abstand voneinander aufweisen, wobei weiter der Freiraum (26) zwischen dem in sich einen geschlossenen Behälter darstellenden Temperaturspeicher (14) und der Außenhülle (12) mit einem Temperatur-Puffermaterial gefüllt ist, dadurch gekennzeichnet, daß die Hülle (18, 20) des Temperaturspeichers (14) und die Außenhülle (12) durchsichtig sind und das Temperaturspeichermedium bei einer Änderung seines Aggregatzustandes eine sichtbare optische Veränderung erfährt.

2. Packung nach Anspruch 1, dadurch gekennzeichnet, daß die sichtbare optische Veränderung in einem Umschlag von opak in transparent besteht.

3. Packung nach Anspruch 1 oder 2, dadurch gekennzeichnet, daß das Puffermaterial ein Wassergel ist.

4. Packung nach Anspruch 3, dadurch gekennzeichnet, daß das Wassergel eingefärbt ist.

5. Packung nach Anspruch 4, dadurch gekennzeichnet, daß die Einfärbung bei der Kältepackung bläulich, bei der Wärmepackung rötlichhautfarben ist.

**Revendications**

1. Plaque pour le traitement thérapeutique de patients par application ou extraction d'énergie avec ou sans effet sur les métabolites, laquelle plaque (10) comporte une enveloppe externe (12) étanche à l'eau et lavable, fermée hermétiquement de tous côtés et stable par rapport à la température, et un accumulateur de température (14), situé dans ladite enveloppe extérieure, qui est divisé en compartiments individuels ou coupelles (22) qui sont remplies d'une matière d'emmagasinage de température et sont espacées les unes des autres, l'espace libre (26) situé entre l'accumulateur de température (14), représentant en soi un compartiment fermé, et l'enveloppe externe (12) étant rempli d'une matière-tampon de répartition de température, caractérisée en ce que l'enveloppe (18, 20) de l'accumulateur de température (14) et l'enveloppe externe (12) sont transparentes et la matière d'emmagasinage de température subit une modification optique visible lorsque son état d'agrégation se modifie.

2. Plaque selon la revendication 1, caractérisée en ce que la modification optique visible consiste en un passage de l'état opaque à l'état transparent.

3. Plaque selon la revendication 1 ou 2, caractérisée en ce que la matière-tampon est un gel à l'eau.

4. Plaque selon la revendication 3, caractérisée en ce que le gel à l'eau est coloré.

5. Plaque selon la revendication 4, caractérisée en ce que la coloration est bleuâtre dans le cas d'une plaque frigorifique et rougeâtre couleur peau dans le cas d'une plaque calorifique.

**Claims**

1. Pack for the remedial treatment of patients by means of releasing or withdrawing energy, with or without the effect of an active substance, the pack (10) having a water-tight and washable outer cover (12), which is temperature-resistant and tightly sealed on all sides, and a temperature store (14) located in the outer cover, which temperature store is subdivided into individual containers or dishes (22) which are filled with a temperature store medium and are spaced apart, the free space (26) between the temperature store (14), which in itself constitutes a closed container, and the outer cover (12) being filled with a temperature buffer material, characterized in that the cover (18, 20) of the temperature store (14) and the outer cover (12) are transparent and the temperature store medium undergoes a visible, optical change when there is a change in its state of aggregation.

2. Pack according to Claim 1, characterized in that the visible, optical change consists in a changeover from opaque to transparent.

3. Pack according to Claim 1 or 2, characterized in that the buffer material is an aqueous gel.

4. Pack according to Claim 3, characterized in that the aqueous gel is coloured.

5. Pack according to Claim 4, characterized in that the colouring of the cold pack is bluish and that of the hot pack is reddish skin-coloured.

FIG. 1

FIG. 2

EP 0 247 037 B1